Europäisches Patentamt

**European Patent Office**

**Office européen des brevets**

(19)

(11) Publication number: **0 029 526**

**A1**

(12) **EUROPEAN PATENT APPLICATION**

(21) Application number: **80106742.2**

(22) Date of filing: **03.11.80**

(51) Int. Cl.³: **A 61 M 1/03**

(30) Priority: **15.11.79 IT 4351579**
**16.09.80 IT 6842180**

(43) Date of publication of application:
**03.06.81 Bulletin 81/22**

(84) Designated Contracting States:
**AT BE CH DE FR GB LI NL SE**

(71) Applicant: **S.p.a. "S.I.F.RA."**
**Località Cà Magre 41**
**I-37063 Isola Della Scala (Verona)(IT)**

(72) Inventor: **Buoncristiani, Umberto**
**Via Brufani 23**
**I-06100 Perugia(IT)**

(74) Representative: **Bongiovanni, Guido et al,**
**c/o Ingg. Carlo e Mario Torta Via Viotti 9**
**I-10121 Torino(IT)**

(54) **Process and device for peritoneal dialysis.**

(57) A process for peritoneal dialysis, whose main characteristic consists in comprising: a first phase during which an end of a first tube (2) branched from a pocket (1) containing a dialyzing solution, and an end portion (35) of a second tube (24) containing a disinfecting liare connected to one another; a second phase during which the dialyzing solution contained in the peritoneal cavity of the patient is allowed to flow through the said second tube (24) and through a third tube communicating with the said first (2) and second (24) tubes; a third phase during which the dialyzing solution contained in the pocket (1) is introduced into the peritoneal cavity; and a fourth phase during which the said first (2) and second (24) tubes are disconnected and the said end portion (35) is filled with disinfecting liquid and closed hermetically.

./...

Fig.15

PROCESS AND DEVICE FOR PERITONEAL DIALYSIS

The present invention relates to a process for the peritoneal dialysis and to a device for carrying out the said process.

As is known, the process followed at present time for carrying out the peritoneal dialysis comprises substantially:

- a first phase during which a tube extending from a pocket containing a dialyzing solution is connected to a free end of a catheter whose opposed end is contained within the peritoneal cavity of the patient;

- a second phase during which the dialyzing solution contained in the pocket is introduced, through the said tube and catheter, into the peritoneal cavity;

- a third phase during which the pocket, already empty, is being worn by the patient awaiting the solution to receive the salts from the blood-vessels by means of the peritoneal membrane;

- a fourth phase during which the dialyzing solution contained in the peritoneal cavity and containing the said salts is made to flow into the said pocket; and

- a fifth phase during which the connection between the tube and the catheter is discontinued and the cycle of dialysis may be resumed by repeating in the same order the phases from one to four described hereinabove.

The process used at present time and described hereinabove in its main phases, has some disadvantages.

In particular, with the said process the risk of infection is high, because, as the end of the catheter to be connected to the tube of the pocket is easily contaminable by the bacteria which are present in surrounding ambient, the flow of the dialyzing solution from the pocket towards the peritoneal cavity involves the introduction into this latter of the bacteria which are present along the connection end of the catheter. This contamination could also give rise to a peritonitis in the patient. In order to reduce as much as possible the risk of infection the pocket is utilized also for the discharge of the dialyzing solution from the peritoneal cavity without disconnecting the pocket from the catheter during the dialysis. Thus, the patient must constantly wear the pocket, but this solution of the problem is not welcome by the patient because of the trouble which it involves.

It is an object of the present invention to provide a process for the peritoneal dialysis and a device for carrying out the process, which will be free from the disadvantages mentioned hereinabove.

The said object is attained by the present invention by means of a process for the peritoneal dialysis, characterized in comprising:

- a first phase during which a first end of a first tube communicating by means of a second end with the interior of a pocket containing a dialyzing solution is connected to a first end portion of a second tibe having a second and opposed end portion communicating with the peritoneal cavity of a patient; the flow along the said first and second tubes being discontinued by first and second interception means respectively, and the said first and second end portions of the said second tube containing, respectively, a disinfecting liquid and a part of a dialyzing solution contained in the said peritoneal cavity;

- a second phase during which the flow inside the said second tube is enabled so that the said dialyzing solution, contained in the said peritoneal cavity, and the said disinfecting liquid contained in the said first end portion of the said second tube will flow towards the outside through the said second tube and a third tube communicating with the said first and second tubes between the said first and second interception means;

- a third phase which takes place at the end of the flow of the said dialyzing solution from the said peritoneal cavity and during which the flow along the said third tube is discontinued and then the flow along the said first tube is allowed to take place so that the said dialyzing solution contained in the said pocket will be introduced into the said peritoneal cavity through the said second tube;

- a fourth phase during which, in order, the flow along the said second tube is interrupted, the said first tube is detached from the said second tube, the said first end portion of the said second tube is filled with disinfecting liquid, and the said first end portion of the said second tube is sealed by means of a plug.

According to the present invention there is also provided a device for carrying out the said process, thes said device being of the type comprising a pocket containing a dialyzing solution and a first tube which communicates with the interior of the said pocket, the said device being characterized in comprising a second tube having a first end portion apt to be detachably connected to a first end of the said first tube, and a second end portion connected in a shrunk fashion to a first end portion of a catheter having a second opposed end portion communicating with the peritoneal cavity of the patient; and a third tube communicating with the said first and second tubes, the said second tube having second interception means disposed thereon.

For a better understanding of the present invention the main phases of the process according to the invention and the most important characteristics of a device for carrying out the said process will now be described in detail with reference to the accompanying drawings, in

which:

FIGURE 1 is a view of a device for carrying out the process according to the invention;

FIGURE 2 is an enlarged and partially sectional view of a detail of the device shown in Fig. 1;

FIGURE 3 is a view of a tube connected to a catheter coming out from the abdomen of a patient;

FIGURE 4 is a view of the tube shown in Fig. 3, connected to the detail shown in Fig. 2;

FIGURE 5 is an enlarged and partially sectional view of the connection between the tube shown in Fig. 3 and the detail of Fig. 2 shown in Fig. 4;

FIGURES from 6 to 14 show sequentially the main phases of the process carried out according to the teachings of the present invention; and

FIGURE 15 is a view of a variant of the device shown in the preceding Figures.

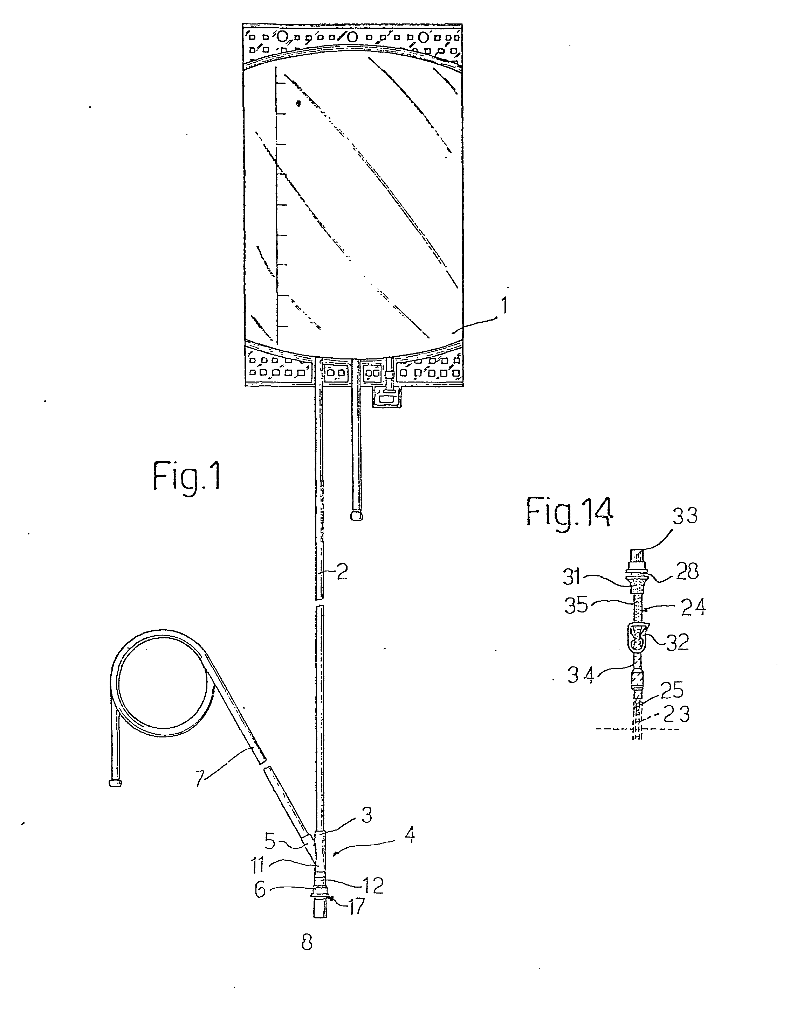

As shown in Fig. 1, reference number 1 indicates a pocket containing, during the use, a dialyzing solution apt to be introduced into a peritoneal cavity. Connected to the pocket 1 is a tube 2 whose end portion is connected to an end portion 3 of a three-way tubular connector 4;

this latter has also a second end portion 5 connected to a tube 7, as well as a third end portion 6 closed by a plug 8.

Portions 3 and 6 are coaxial to one another, whilst the axis of the portion 5 is convergent with the axis defined by the portions 3 and 6. Finally, the tube 7 has its own free end conveniently closed by welding.

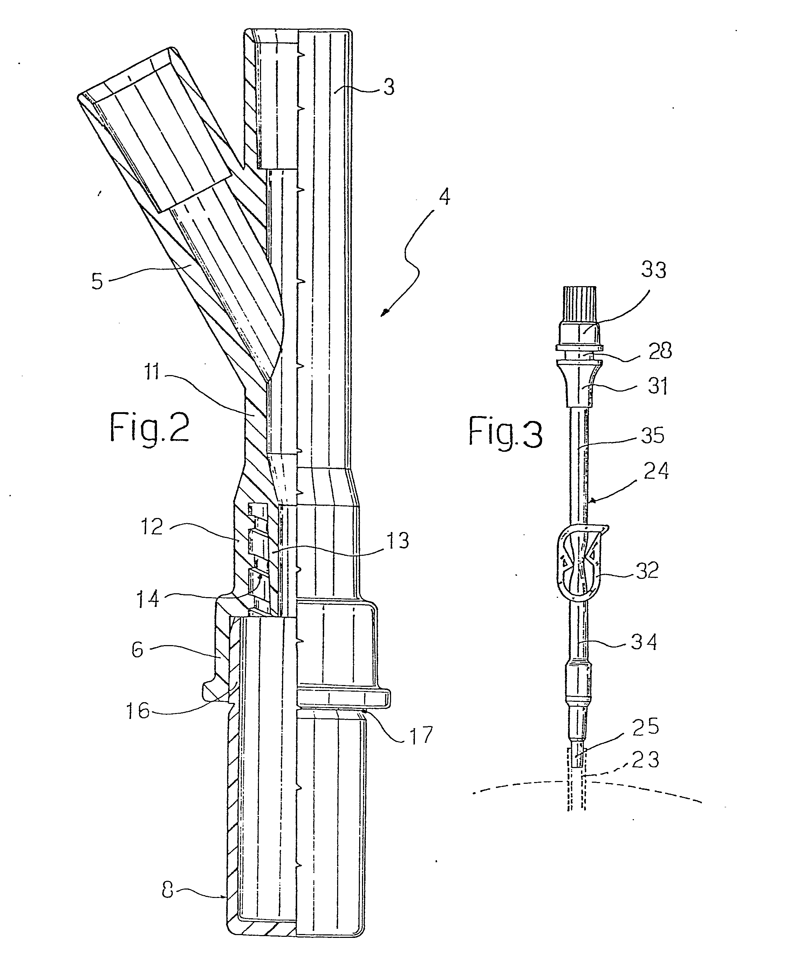

With particular reference to Fig. 2, the tubular connector 4 has a central cylindrical portion situated in an intermediate position between the portions 3 and 5 and the portion 6. Extending coaxially from the portion 11 towards the end portion 6 is a pair of concentric rings 12, 13, of which the ring having a larger diameter and indicated by reference numeral 12 has an inner threaded portion 14.

Connected to the ring 12 is the said end portion 6 whose diameter is larger than the diameter of the ring 12. Fixed inside the portion 6, for example bey means of an adhesive, is a cylindrical end portion 16 of the plug 8 which has on its outer surface an annular prebreaking groove 17 adjacent the portion 6 of the connector 4.

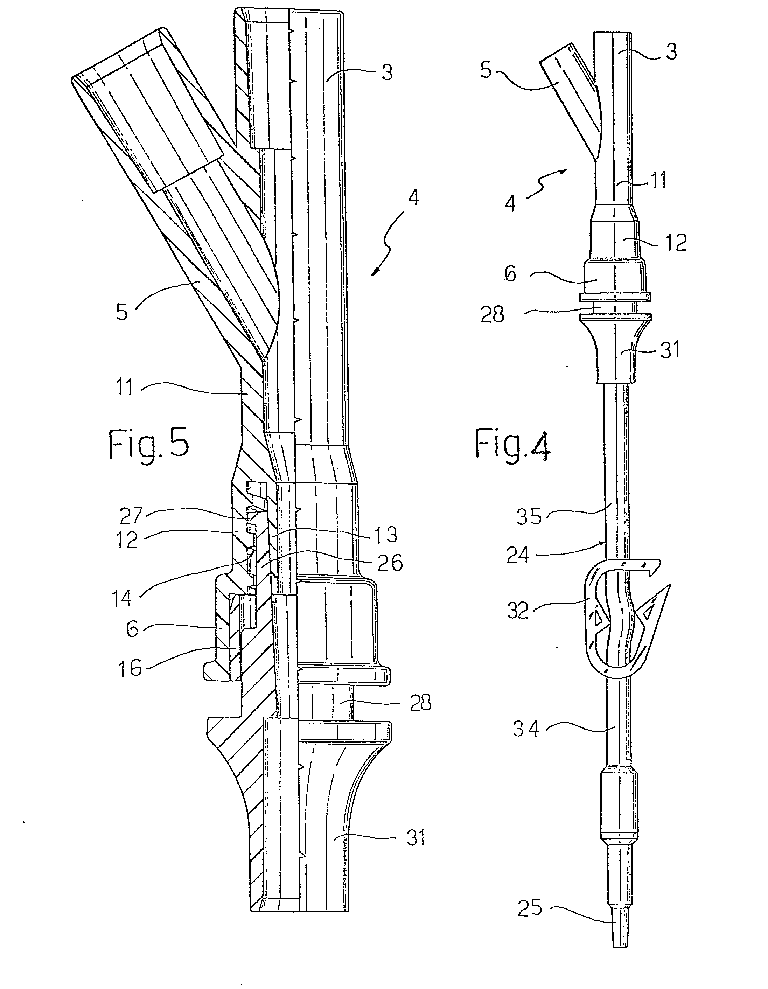

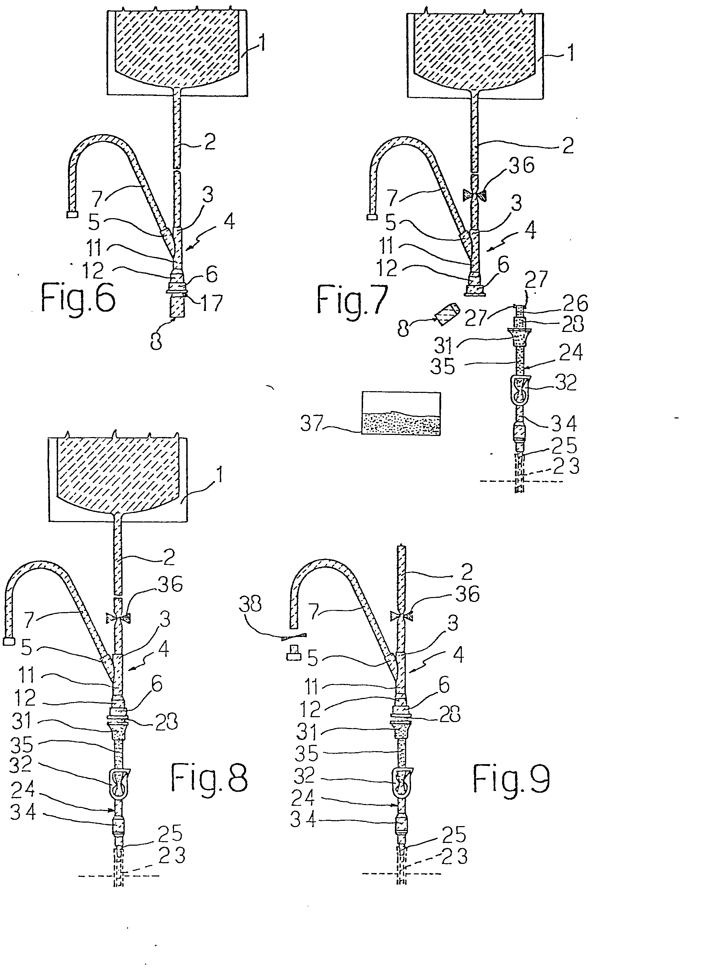

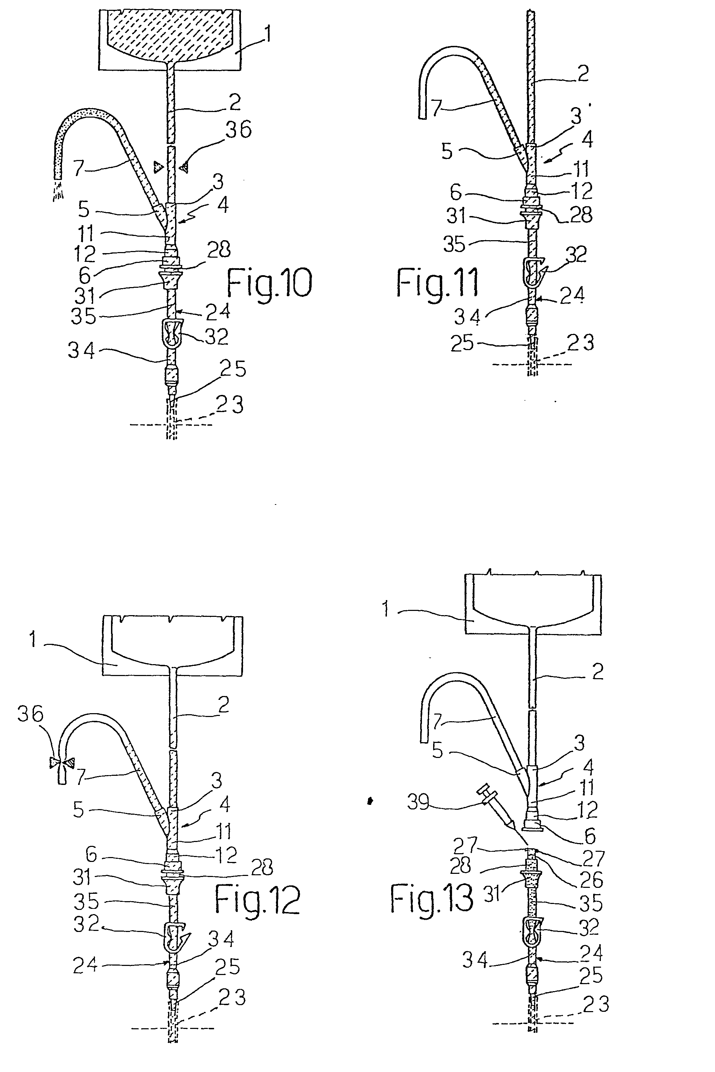

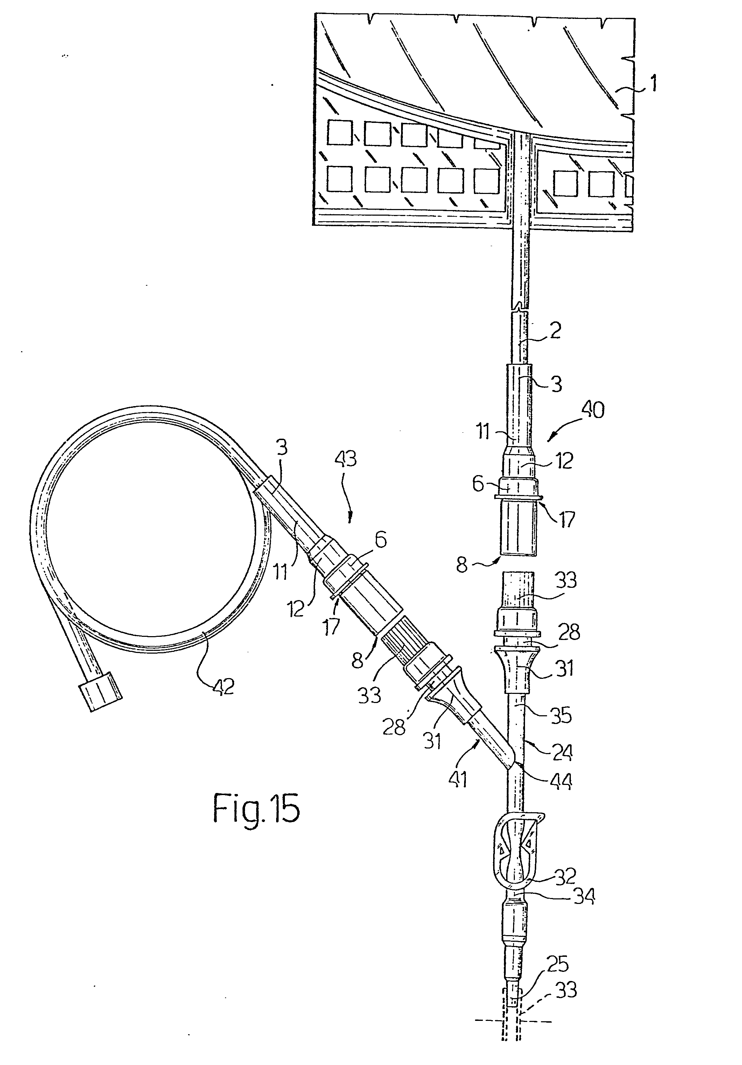

As shown in Fig. 3, reference numeral 23 indicates a catheter, an end portion (not shown) of which is situated in the peritoneal cavity of a patient.In use, catheter 23 is connected to a tube 24; in particular (see Figures 3 and 4), the tube 24 has a conical end 25 which is mounted in a shrunk fashion within the catheter itself. Referring now to Fig. 5, the tube 24 has, at the portion opposed to the end 25, an end 26 provided with diametrically opposed projections 27 each of which is apt to cooperate,in use, with the threaded surface 14 of the ring 12. Adjacent the end 26 the tube 24 aa a portion 28 whose outer diameter is slightly smaller than the inner diameter of the portion 16 of the plug 8 in order to establish a sealing connection between the tube 24 and the tubular connector 4. A contribution to the said seal is given also by the forced cooperation which takes place between the inner surface of the end 26 and the facing outer surface of the ring 13 (Fig.5). Adjacent the portion 28 the tube 24 has a conical portion which forms a convenient handle and facilitates the
connection and disconnection operations between the tube
24 and the connector 4. In addition, disposed along the
tube 24 are pliers 32 of the "klemmer" type, apt to intercept the flow of the dialyzing solution along the said tube
24. During the phases of the process according to the present
invention in which there is no connection between the tube
24 and the connector 4, the tube 24 is provided with a
sealing plug 33 which is threaded internally. In such phases
of the process the pliers 32 interrupt the flow along the
tube 24 so as to divide the tube in two parts, the part
which communicates with the catheter 23 being indicated by
reference numeral 34 and containing a part of the dialysing
solution contained in the peritoneal cavity, whils the part
which is closed by the plug 33 is indicated by reference
numeral 35 and preferably contains a disinfecting liquid.

Referring now to Figures from 6 to 14, the process for
the peritoneal dialysis according to the teachings of the
present invention comprises substantially the following
phases:

- a first phase during which the flow along the tube 2
of the dialyzing solution contained in the pocket 1 is
interrupted by means of pliers 36 so that during the detachment of the outer portion of the plug 8 from the connector 4 the said solution will not flow out;

- a second phase during which the connector 4 is first immersed into a tank 37 containing a disinfecting liquid and then connected to the end 26 of the tube 24 already connected to the catheter 23 and having the pliers 32 in the closure position;

- a third phase during which a cut is made by means of scissors near the welded end of the tube 7 so that by allowing the flow to take place for a limited pre-established time along the tube 2 a washing of the tube 7 is obtained;

- a fourth phase during which the solution is allowed to flow along the tube 24, so that the dialyzing solution contained in peritoneal cavity and the disinfecting liquid present inside the portion 34 of the tube 24 and the portion 6 of the connector 4 are made to flow through the tube 7 into a  special container (not shown);

- a fifth phase during which the flow along the tube 7 is interrupted and subsequently the the flow along the tube 2 is allowed to take place, so that the dialyzing solution contained in the pocket 1 is introduced into the peritoneal cavity; and

- a sixth phase during which, in order, the flow along tube 24 is interrupted, connector 4 is detached from the tube 24, and, for example by means of a syringe 39, the part 35 of the tube 24 is filled with a disinfecting liquid and finally the end of the portion 35 is sealed by means of the plug 33.

Figure 15 shows a variant of the device for the peritoneal dialysis described in relation to Figures from 1 to 5. According to this variant the device comprises the pocket 1 connected to the tube 2, whose end portion is connected to a two-way connector 40, which only differs from the connector 4 (shown in Fig. 2) in that the end portion 5 is missing. For this reason the constructional elements of the connector 40 which are similar to the constructional elements of the conccetor 4 are indicated by the same reference numerals. In addition, the device shown in Fig. 15 is provided with the tube 24 from which, however, upstream of the pliers 32, a tube 41 is branched whose free end is shaped like the welded end of the tube 7 (Fig. 1) and a second end of which is connected to a connector 43 similar to connector 40. The said tube 40 is intended to faciltate the discharge operations of the dialyzing solution from the peritoneal cavity. Referring to Fig. 15, a part 44 is defined which is apt to contain a disinfecting liquid and comprises the part 35 of the tube 24 and the tube 41 itself.

The univocality of the process according to the present invention remaining unchanged, the substantial difference between the two devices described herein consists in that in the first device (Figures from 1 to 5) the discharge tube (indicated by reference numeral 7) is branched from the tube 2, whilst in the second device (Fig. 15) the discharge tube, indicated by reference numeral 41, is branched from the tube 24.

The process realized in accordance with the teachings of the present invention has numerous advantages.

In particular, with the said process a sterilization downstream of the possible contamination is ensured, inasmuch as the connection between the connector 4, 40 and the tube 24 is made exclusively in the presence of disinfecting liquid; therefore, the risk of infection is drastically reduced. Moreover, in the process of the invention the discharge of the dialyzing solution from the peritoneal cavity towards the outside is carried out after the connection between the connector 4,40 and the tube 24 has been established, so that an additional washing is carried out. The patient is not compelled to wear constantly the pocket 1, but only the tube 24 which may be of reduced dimensions and therefore is not troublesome for the patient; moreover, the tube 24 may be utilized for very long periods of time. Finally, the process according to the invention allows a simplification of the peritoneal dialysis technique, as well as a reduction of the time of application of the device for carrying out the process.

Finally, it is clear that modifications and variations may be made to the phases of the process and the characteristics of the device for carrying out the said process, without departing from the scope of the invention.

C L A I M S

1.- A process for the peritoneal dialysis, characterized in comprising:

- a first phase during which a first end of a first tube (2) communicating by means of a second end with the interior of a pocket (1) containing a dialysing solution is connected to a first end portion (35) of a second tube (24) having a second and opposed end portion (34) communicating with the peritoneal cavity of a patient; the flow along the said first (2) and second (24) tubes being discontinued by first (36) and second (32) interception means respectively, and the said first and second end portions (35, 34) of the said second tube (24) containing, respectively, a disinfecting liquid and a part of a dialyzing solution contained in the said peritoneal cavity;

- a second phase during which the flow within the said second tube (24) is enabled so that the said dialyzing solution, contained in the said peritoneal cavity, and the said disinfecting liquid contained in the said first end portion (35) of the said second tube (24) will flow towards the outside through the said second tube (24) and a third tube (7, 41) which communicates with the said first (2) and second (24) tubes between the said first (36) and second (32) interception means;

- a third phase which takes place at the end of the flow of the said dialyzing solution from the said peritoneal cavity and during which the flow along the said third tube (7, 41) is discontinued and then the flow along the said first tube (2) is allowed to take place so that the said dialyzing solution contained in the said pocket (1) will be introduced into the said peritoneal cavity through the said sec..d tube (24);

- a fourth phase during which, in order, the flow along the said second tube (24) is interrupted, the said first tube (2) is detached from the said second tube (24), the said first end portion (35) of the said second tube (24) is filled with a disinfecting liquid, and the said first end portion (35) of the said second tube (24) is sealed by means of a plug (33).

2.- A process as claimed in Claim 1, characterized in comprising between the said first phase and the said second phase an intermediate phase during which the flow along the said first tube (2) is enabled for a pre-established period of time  so that a determined  quantity of the said dialyzing solution contained in the pocket (1) will flow towards the outside through the said third tube (7, 41).

3.- A process as claimed in at leas one of the Claims 1 and 2, characterized in that during the said first phase the said first end of the said first tube (2) is made to contact the disinfecting liquid before being connected to the said first end portion (35) of the said second tube (24).

4.- A device for carrying out the process as claimed in any one of the Claims from 1 to 3, and of the type which comprises a pocket (1) containig a dialyzing solution, and a first tube (2) which communicates with the interior of the said pocket (1), characterized in comprising a second tube (24) having a first end portion (35) apt to be detachably connected to a first end of the said first tube (2), and a second end portion (25) connected in a shrunk fashion to a first end portion of a catheter (23) having a second opposed end portion communicating with the peritoneal cavity of the patient; and a third tube (7, 41) which communicates with the said first (2) and second (24) tubes, the said second tube having second interception means (32) disposed thereon.

5.- A device as claimed in Claim 4, characterized in that the said first end portion (35) of the said second tube (24) is provided with connection means (27) arranged to engage the said first end of the said first tube (2).

6.- A device as claimed in Claim 5, characterized in that . . . the said first end portion (35) of the said second tube (24) has a cross-section stepwise decreasing towards
the outside which defines a tubular head portion (26) having
a smaller cross-section and an intermediate portion having
a larger cross-section, whose inner and outer surfaces
respectively are apt to cooperate in a sealing fashion with
corresponding surfaces formed in the said first end of the
said first tube (2).

7.- A device as claimed in any one of the Claims from
4 to 6, characterized in that the said second end portion
(25) of the said second tube (24) is conical and tapered
towards the ouside and is apt to be mounted in a shrunk
fashion in the said first end portion of the said
catheter (23).

8.- A device as claimed in any one of the Claims from
4 to 7, characterized in that the said third tube (7) is
branched from the the said first tube (2) downstream of
first interception means (36) disposed along the said first
tube (2).

9.- A device as claimed in any one of the Claims from
4 to 7, characterized in that the said third tube (41) is
branched from the said second tube (24) upstream of the said
second interception means (32).

10.- A device as claimed in Claim 9, characterized in that an end portion of the said third tube (41) is provided with connection means arranged to engage an end of a fourth tube (42) apt to facilitate the discharge of the dialyzing solution from the peritoneal cavity.

11.- A device as claimed in Claims 9 or 19, characterized in that the said end portion of the said third tube (41) is provided with a sealing plug (33).

**Fig.1**

**Fig.14**

Fig.2

Fig.3

Fig.5

Fig.4

Fig.6

Fig.7

Fig.8

Fig.9

Fig.10

Fig.11

Fig.12

Fig.13

Fig.15

0029526

6/6

European Patent Office

**EUROPEAN SEARCH REPORT**

Application number

EP 80 10 6742

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | <u>DE - A - 2 809 303</u> (POPOVICH)<br><br>* Page 1, line 1 - page 2, line 17; page 9, line 11 - page 16, line 2 *<br><br>-- | 1-11 | A 61 M 1/03 |
| | <u>US - A - 3 545 438</u> (DE VRIES)<br><br>* Abstract; column 2, line 55 - column 4, line 42 *<br><br>-- | 1 | |
| A | <u>FR - A - 2 440 740</u> (ABG-SEMCA)<br><br>* Page 20, line 1 - page 23, line 32; claims 1-14 *<br><br>---- | 1 | **TECHNICAL FIELDS SEARCHED (Int. Cl.³)**<br><br>A 61 M |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| | | | |
|---|---|---|---|
| X | The present search report has been drawn up for all claims | | |
| Place of search<br>The Hague | Date of completion of the search<br>26-01-1981 | Examiner<br>PESCHEK | |

EPO Form 1503.1  06.78